# EUROPEAN PATENT APPLICATION

(11) **EP 4 046 565 A1**
(43) Date of publication of application: **24.08.2022**
(21) Application number: 20876707.9
(22) Date of filing: 16.10.2020
(51) Int. Cl.: A61B 1/05

(54) **ENDOSCOPE CAMERA AND ENDOSCOPE CAMERA SYSTEM**

(30) Priority: 16.10.2019 CN 201910983275
(71) Applicant: Shenzhen Mindray Bio-Medical Electronics Co., Ltd., Shenzhen, Guangdong 518057 (CN)
(72) Inventor: ZENG, Qiang, Shenzhen, Guangdong 518057 (CN)
(74) Representative: KIPA AB
(86) International application number: PCT/CN2020/121638
(87) International publication number: WO 2021/073619

(57) **Abstract**

An endoscope camera (50) and an endoscope camera system (1000), the endoscope camera (50) comprising a handle (1), a chip module (2), an optical module (3) and a hand wheel (4), the chip module (2) being mounted in an accommodation cavity (11) of the handle (1), the chip module (2) being used to convert an optical signal into an electrical signal, and the optical module (3) being connected to the chip module (2). Given that the chip module (2) is directly connected to the optical module (3), and there are no other components between the optical module (3) and the chip module (2), a dimensional chain calculation is shorter, lowering the influence of machining and mounting errors, and thereby improving optical imaging quality; in addition, the optical module (3) and the chip module (2) being directly connected shrinks an axial dimension of the entire endoscope camera (50), realizing miniaturization of the endoscope camera (50).

## Description

### TECHNICAL FIELD

The disclosure relates to the technical field of in-vivo diagnostic instrument, and more particularly to an endoscope camera and an endoscope camera system.

### BACKGROUND

Hard endoscope, such as cystoscope and hysteroscope, is mainly used for the diagnosis and/or treatment of lesions in the natural lumen of the superficial parts of the human body and the opened lumen through puncture. The hard endoscope cannot be bent during operation.

Hard tube endoscope mainly includes a camera, a light source, light guide bundles, a hard endoscope, an optical bayonet, a camera host and a displayer. The camera includes an optical module, a chip module and other components. The components inside the camera need to be installed in alignment with each other along the optical axis. Each component has its own processing error and there are still installation errors between components. Therefore, reducing the alignment error is an important factor to ensure the optical imaging. However, in the prior art, there are many components and connection relationships in the camera, and the calculation of the dimension chain is too long, which makes it difficult to ensure the alignment accuracy, so it is difficult to ensure the optical imaging quality of the camera.

### SUMMARY

In one embodiment, an endoscope camera is provided, which including:
a handle which is provided with an accommodation cavity, wherein an end of the handle is provided with an opening which is communicated with the accommodation cavity;
a chip module which includes a housing and a chip assembly, wherein the housing is installed inside the accommodation cavity of the handle, the chip assembly is installed inside the housing, wherein the housing is provided with a light inlet end at which a light inlet is arranged, wherein the chip assembly is operable to convert an optical signal into an electrical signal;
an optical module which is connected with the chip module, wherein the optical module includes a lens barrel, a fixed optical assembly and an adjustable optical assembly, wherein the fixed optical assembly is installed at one end of the lens barrel which is away from the chip module, and the adjustable optical assembly is axially and movably installed inside the lens barrel; and
a hand wheel which is rotatably sheathed outside the lens barrel and connected with the adjustable optical assembly through a connection element, wherein the hand wheel is operable to adjust an axial position of the adjustable optical assembly.

In one embodiment, the other end of the lens barrel is connected with the light inlet end of the housing.

In one embodiment, the light inlet end of the housing is provided with an installation base, wherein the installation base is provided with an installation hole into which the other end of the lens barrel is inserted, wherein the lens barrel is axially aligned with the light inlet.

In one embodiment, a first connection part is provided inside the installation hole of the installation base, a second connection part is provided at the other end of the lens barrel, wherein the first connection part is connected with the second connection part.

In one embodiment, the first connection part is an internal thread and the second connection part is an external thread; or the first connection part is an engaging groove, and the second connection part is an engaging element.

In one embodiment, the installation hole of the installation base is further provided with a first positioning part which is arranged axially with the first connection part side by side, the other end of the lens barrel is provided with a second positioning part which is arranged axially with the second connection part side by side, and the first positioning part is positionally limited on the second positioning part.

In one embodiment, the first positioning part is a plurality of axial protrusions, and the second positioning part is an annular surface; or the first positioning part is an annular surface, and the second positioning part is a plurality of axial protrusions.

In one embodiment, the installation base is provided with a locking hole, inside which a locking screw is installed, and the locking screw locks the other end of the lens barrel inside the installation base.

In one embodiment, the lens barrel of the optical module and the housing of the chip module are an integrated structure.

In one embodiment, the opening, at the end of the handle which is adjacent to the hand wheel, is provided with a front cover, wherein the front cover is provided with a through hole, wherein the lens barrel penetrates into and is arranged inside the through hole, the housing is connected with the front cover, and the front cover is operable to install the chip module and the optical module into the accommodation cavity of the handle.

In one embodiment, the lens barrel is provided with a position-limiting part which is operable to limit an axial position of the lens barrel.

In one embodiment, the position-limiting part is clamped between the front cover and the installation base.

In one embodiment, the position-limiting part is an annular protrusion or a plurality of radial protrusions arranged at a same circumference.

In one embodiment, the endoscope camera further includes a fixing support which is positioned inside the accommodation cavity of the handle, one end of the fixing support is connected with the front cover, and the other end of the fixing support is connected with the housing of the chip module.

In one embodiment, the fixing support has an L-shaped structure and includes a long arm and a short arm which are perpendicular to each other, the long arm is attached to the installation base and the housing and connected with the housing, and the short arm is attached to the front cover and connected with the front cover.

In one embodiment, the long arm and the short arm of the fixing support are respectively fixed with the housing and the front cover through a screw.

In one embodiment, the endoscope camera comprises a plurality of fixing supports, and at least one of the plurality of fixing supports is connected with a grounded cable.

In one embodiment, the fixing support which is connected with the grounded cable is a grounded support, and an end of the long arm of the grounded support extends to an axial end of the housing.

In one embodiment, an endoscope camera is provided, which including:
a handle which is provided with an accommodation cavity, wherein an end of the handle is provided with an opening which is communicated with the accommodation cavity;
a chip module which is installed inside the accommodation cavity of the handle and operable to convert an optical signal into an electrical signal;
an optical module which is directly connected with the chip module and exposed entirely or partially from an opening of the handle; and
a hand wheel which is rotatably sheathed outside the optical module.

In one embodiment, the chip module is provided with an installation base, wherein the installation base is provided with an installation hole into which an end of the optical module is inserted and installed.

In one embodiment, a first connection part is provided inside the installation hole of the installation base, a second connection part is provided at the end of the optical module, wherein the first connection part is connected with the second connection part.

In one embodiment, the installation hole of the installation base is further provided with a first positioning part which is arranged axially with the first connection part side by side, one end of the optical module is provided with a second positioning part which is arranged axially with the second connection part side by side, and the first positioning part is positionally limited on the second positioning part.

In one embodiment, an endoscope camera is provided, which including:
a handle which is provided with an accommodation cavity, wherein an end of the handle is provided with an opening which is communicated with the accommodation cavity;
a chip module which includes a housing and a chip assembly, wherein the housing is installed inside the accommodation cavity of the handle, the chip assembly is installed inside the housing, wherein the housing is provided with a light inlet end at which a light inlet is arranged; and
an optical module which includes a lens barrel and an optical assembly, wherein an end of the lens barrel is connected with the light inlet end of the housing, and the optical assembly is installed inside the lens barrel and operable to transmit an optical signal.

In one embodiment, the housing is provided with an installation base, wherein the installation base is provided with an installation hole into which the end of the lens barrel is inserted.

In one embodiment, a first connection part is provided inside the installation hole of the installation base, a second connection part is provided with the end of the lens barrel, wherein the first connection part is connected with the second connection part.

In one embodiment, the installation hole of the installation base is further provided with a first positioning part which is arranged axially with the first connection part side by side, the end of the lens barrel is provided with a second positioning part which is arranged axially with the second connection part side by side, and the first positioning part is positionally limited on the second positioning part.

In one embodiment, the chip module further includes an adjustment element, which is arranged at the housing, for a radial adjustment of the chip assembly.

In one embodiment, the adjustment element includes an adjustment rod, the housing and/or the lens barrel are provided with an adjustment hole, wherein the adjustment rod penetrates into and is arranged inside the adjustment hole, and the adjustment rod is operable to adjust and fix a radial position of the chip assembly and/or a radial position of the optical assembly.

In one embodiment, the light inlet end of the housing is provided with a first connection part, and a light outlet end of the lens barrel is provided with a second connection part; the first connection part is provided with a first adjustment hole, and the second connection part is provided with a second adjustment hole; the adjustment rod is arranged inside the first adjustment hole and the second adjustment hole and operable to fix the first connection part and the second connection part; wherein an inner diameter of the first adjustment hole is adapted to an outer diameter of the adjustment rod, an inner diameter of the second adjustment hole is greater than the outer diameter of the adjustment rod; or an inner diameter of the second adjustment hole is adapted to the outer diameter of the adjustment rod, and an inner diameter of the first adjustment hole is greater than the outer diameter of the adjustment rod; or inner diameters of both the first adjustment hole and the second adjustment hole are greater than the outer diameter of the adjustment rod.

In one embodiment, the light inlet end of the housing is provided with an adjustment base; wherein the adjustment base is provided with a first connection part, and a light outlet end of the lens barrel is provided with a second connection part; the first connection part is provided with a first adjustment hole, and the second connection part is provided with a second adjustment hole; the adjustment rod is arranged inside the first adjustment hole and the second adjustment hole and operable to fix the first connection part and the second connection part; wherein an inner diameter of the first adjustment hole is adapted to an outer diameter of the adjustment rod, an inner diameter of the second adjustment hole is greater than the outer diameter of the adjustment rod; or an inner diameter of the second adjustment hole is adapted to the outer diameter of the adjustment rod, and an inner diameter of the first adjustment hole is greater than the outer diameter of the adjustment rod; or inner diameters of both the first adjustment hole and the second adjustment hole are greater than the outer diameter of the adjustment rod.

In one embodiment, the light inlet end of the housing is provided with an installation hole, and an end of the adjustment base can be axially and adjustably installed inside the installation hole of the housing.

In one embodiment, the housing is provided with a fixing screw which fixes an end of the adjustment base to the housing.

In one embodiment, a front cover is installed at an opening of the accommodation cavity of the handle, a through hole is arranged at a middle of the front cover, and the lens barrel penetrates into and is arranged inside the through hole; wherein an axial inner side surface of the front cover is provided with a screw hole, the adjustment rod is a bolt or a screw, and the adjustment rod is connected inside the screw hole of the front cover.

In one embodiment, the light inlet end of the housing is provided with an installation hole and an adjustment base; wherein an end of the adjustment base is installed inside the installation hole of the housing and has an outer diameter which is smaller than an inner diameter of the installation hole of the housing; wherein at least three adjustment holes are arranged at one circumference of the light inlet end of the housing, and each adjustment hole is provided with one adjustment rod.

In one embodiment, the adjustment hole is a screw hole, the adjustment rod is a bolt or a screw, and an end of the adjustment rod penetrates into the adjustment hole and abuts against the end of the adjustment base.

In one embodiment, a gasket is arranged between the end of the adjustment rod and the adjustment base.

In one embodiment, the optical module further includes an adjustment element, which is arranged at the lens barrel, for a radial adjustment of the chip assembly.

In one embodiment, the adjustment element includes an adjustment rod, the housing and/or the lens barrel are provided with an adjustment hole, wherein the adjustment rod penetrates into and is arranged inside the adjustment hole, and the adjustment rod is operable to adjust and fix a radial position of the chip assembly and/or a radial position of the optical assembly.

In one embodiment, the light inlet end of the housing is provided with a first connection part, and a light outlet end of the lens barrel is provided with a second connection part; the first connection part is provided with a first adjustment hole, and the second connection part is provided with a second adjustment hole; the adjustment rod is arranged inside the first adjustment hole and the second adjustment hole and operable to fix the first connection part and the second connection part; wherein an inner diameter of the first adjustment hole is adapted to an outer diameter of the adjustment rod, an inner diameter of the second adjustment hole is greater than the outer diameter of the adjustment rod; or an inner diameter of the second adjustment hole is adapted to the outer diameter of the adjustment rod, and an inner diameter of the first adjustment hole is greater than the outer diameter of the adjustment rod; or inner diameters of both the first adjustment hole and the second adjustment hole are greater than the outer diameter of the adjustment rod.

In one embodiment, a light outlet end of the lens barrel is provided with an adjustment base; wherein the adjustment base is provided with a first connection part, and a light inlet end of the housing is provided with a second connection part; the first connection part is provided with a first adjustment hole along its circumference, and the second connection part is provided with a second adjustment hole; an inner diameter of one of the first adjustment hole and the second adjustment hole is adapted to an outer diameter of the adjustment rod, and an inner diameter of the other one of the first adjustment hole and the second adjustment hole is greater than the outer diameter of the adjustment rod; or inner diameters of both the first adjustment hole and the second adjustment hole are greater than the outer diameter of the adjustment rod; wherein the adjustment rod is arranged inside the first adjustment hole and the second adjustment hole and operable to fix the first connection part and the second connection part.

In one embodiment, a front cover is installed at an opening of the accommodation cavity of the handle, a through hole is arranged at a middle of the front cover, and the lens barrel penetrates into and is arranged inside the through hole; wherein an axial inner side surface of the front cover is provided with a screw hole, the adjustment rod is a bolt or a screw, and the adjustment rod is connected inside the screw hole of the front cover.

In one embodiment, an outer diameter of the optical assembly is smaller than an inner diameter of the lens barrel, at least three adjustment holes are arranged at one circumference of the lens barrel, each adjustment hole is provided with one adjustment rod, the adjustment hole is a screw hole, the adjustment rod is a bolt or a screw, an end of the adjustment rod penetrates into the adjustment hole and abuts against the optical assembly.

In one embodiment, the optical assembly includes a lens base and a lens group, wherein the lens base has a cylindrical structure, the lens group is installed inside the lens base, and the end of the adjustment rod abuts against an outer circumferential surface of the lens base.

In one embodiment, a gasket is arranged between the end of the adjustment rod and the outer circumferential surface of the lens base.

In one embodiment, an endoscope camera is provided, which including:
a handle which is provided with an accommodation cavity, wherein an end of the handle is provided with an opening which is communicated with the accommodation cavity;
a chip module which includes a housing and a chip assembly, wherein the housing is installed inside the accommodation cavity of the handle, the chip assembly is installed inside the housing, wherein the housing is provided with a light inlet end at which a light inlet is arranged, wherein the chip assembly is operable to convert an optical signal into an electrical signal; and
an optical module which includes a lens barrel and an optical assembly, wherein the optical assembly is installed inside the lens barrel, and the lens barrel is provided with a light outlet end which is connected with the light inlet end of the housing;
an end of the housing, which faces the lens barrel, is provided with an installation hole, an end of the lens barrel is arranged inside the installation hole; wherein an installation gap is provided for radial adjustment between the housing and the lens barrel.

In one embodiment, the housing and the lens barrel are fixed by glue or screw.

In one embodiment, the housing is provided with a screw hole, the screw is fixed inside the screw hole of the housing, an end of the screw abuts against an outer circumferential surface of the lens barrel, and a gasket is arranged between the end of the screw and the outer circumferential surface of the lens barrel.

In one embodiment, an endoscope camera is provided, which including:
a handle which is provided with an accommodation cavity, wherein an end of the handle is provided with an opening which is communicated with the accommodation cavity;
a chip module which includes a housing and a chip assembly, wherein the housing is installed inside the accommodation cavity of the handle, the chip assembly is installed inside the housing, wherein the housing is provided with a light inlet end at which a light inlet is arranged, wherein the chip assembly is operable to convert an optical signal into an electrical signal; and
an optical module which includes a lens barrel and an optical assembly, wherein the optical assembly is installed inside the lens barrel, the lens barrel is provided with a light outlet end which is connected with the light inlet end of the housing;
one end of the housing which faces the lens barrel is provided with an installation part, the installation part of the housing is arranged inside the lens barrel, and an installation gap is provided for radial adjustment between the installation part of the housing and the lens barrel.

In one embodiment, the installation part of the housing and the lens barrel are fixed by glue or screw.

In one embodiment, the lens barrel is provided with a screw hole, the screw is fixed inside the screw hole of the lens barrel, an end of the screw abuts against the installation part of the housing, and a gasket is arranged between the end of the screw and the installation part of the housing.

In one embodiment, an endoscope camera system is provided, which including a light source, light guide bundles, an endoscope, an optical bayonet, a communication cable, a camera host, a displayer, a video connection line and the endoscope camera discussed above; wherein the light source is connected with the endoscope through the light guide bundles, and one end of the endoscope camera is connected with the endoscope through the optical bayonet, other end of the endoscope camera is connected with the camera host through the communication cable, wherein the camera host is connected with the displayer through the video connection cable.

Based on the endoscope camera and endoscope camera system according to the above embodiments, because the optical module is directly connected with the chip module and there are no other components between the optical module and the chip module, the calculation of the dimension chain is shorter, which reduces the influence of processing and installation errors, improves the optical imaging quality, and realizes miniaturization of the endoscope camera.

### BRIEF DESCRIPTION OF THE DRAWINGS

FIG. 1 is a structural diagram of an endoscope camera system in an embodiment of this disclosure.
FIG. 2 is a sectional diagram of an endoscope camera in an embodiment of this disclosure.
FIG. 3 is a sectional diagram of an adjustable optical assembly in an embodiment of this disclosure.
FIG. 4 is a sectional diagram of a connection between a chip module and an optical module in an embodiment of this disclosure.
FIG. 5 is a sectional diagram of a connection between a chip module and an optical module in an embodiment of this disclosure.
FIG. 6 is an explosion diagram of a connection between a chip module and an optical module in an embodiment of this disclosure.
FIG. 7 is a sectional diagram of an endoscope camera in an embodiment of this disclosure.
FIG. 8 is a sectional diagram of an endoscope camera in an embodiment of this disclosure.
FIG. 9 is a structural diagram of an endoscope camera in an embodiment of this disclosure.
FIG. 10 is an axially sectional diagram of an endoscope camera in an embodiment of this disclosure.
FIG. 11 is an explosion diagram of a chip module in an embodiment of this disclosure.
FIG. 12 is a structural diagram of a chip module in an embodiment of this disclosure.
FIG. 13 is a partially enlarged diagram of A in FIG. 10.
FIG. 14 is an axially sectional diagram of an endoscope camera in an embodiment of this disclosure.
FIG. 15 is a structural diagram of a support in an embodiment of this disclosure.
FIG. 16 is an axially sectional diagram of a chip module in an embodiment of this disclosure.
FIG. 17 is a radially sectional diagram of a chip module in an embodiment of this disclosure.
FIG. 18 is an axially sectional diagram of an optical module in an embodiment of this disclosure.
FIG. 19 is a radially sectional diagram of an optical module in an embodiment of this disclosure.
FIG. 20 is an axially sectional diagram of an endoscope camera in an embodiment of this disclosure.
FIG. 21 is a radially sectional diagram of an endoscope camera in an embodiment of this disclosure.
FIG. 22 is an axially sectional diagram of an endoscope camera in an embodiment of this disclosure.
FIG. 23 is a radially sectional diagram of an endoscope camera in an embodiment of this disclosure.

### DESCRIPTION OF THE PREFERRED EMBODIMENTS

This disclosure will be further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or features described in the specification may be combined in any appropriate manner to form various embodiments. At the same time, the steps or actions in the method description can also be exchanged or adjusted in order in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc., in the specification are operable to distinguish different objects, rather than to describe a specific order. In addition, the terms "connection", and "linkage" mentioned in this application include direct and indirect connection (linkage), unless otherwise specified.

The preset disclosure will be further described in detail through specific embodiments in combination with the accompanying drawings.

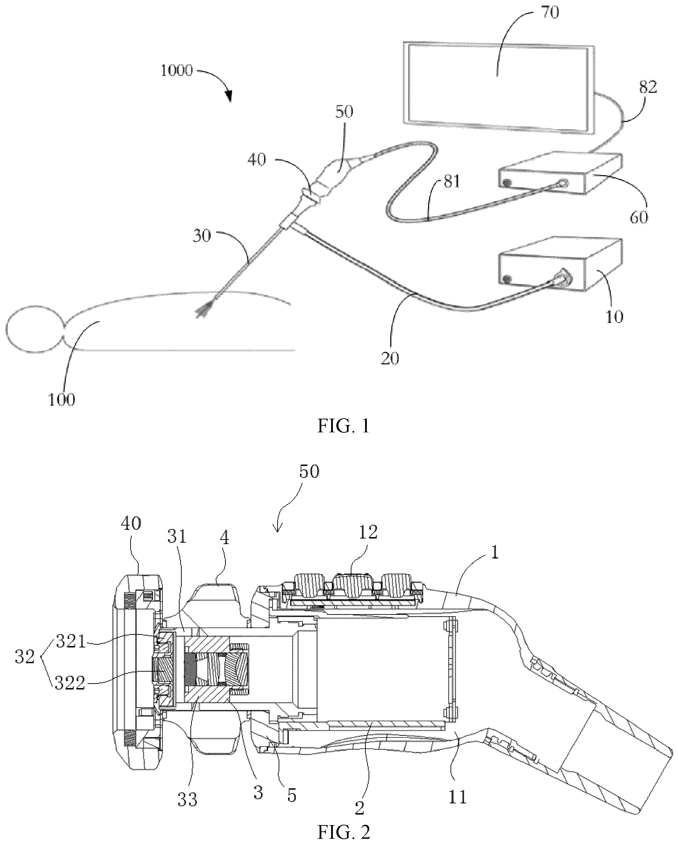

As shown in FIG.1, an endoscope camera system 1000 is provided in one embodiment. The endoscope camera system 1000 includes a light source 10, light guide bundles 20, a hard endoscope 30, an optical bayonet 40, an endoscope camera 50, a communication cable 81, a camera host 60, a displayer 70 and a video connection line 82. The camera host 60 is connected with the endoscope camera 50 through the communication cable 81. In some embodiments, the communication cable 81 may be an optical communication cable, such as an optical fiber. The endoscope camera 50 converts an image signal (electrical signal) into an optical signal, which is transmitted to the camera host 60 through the communication cable 81, and then the camera host 60 converts the optical signal into an electrical signal. The camera host 60 is connected to the displayer 70 through the video connection line 82 for transmitting a video signal to the displayer 70 for display. Those skilled in the art should understand that FIG. 1 is only an example of the endoscope camera system 1000 and does not constitute a limitation on the endoscope camera system 1000. The endoscope camera system 1000 may include more or fewer components than shown in FIG. 1, or combine some components, or different components. For example, the endoscope camera system 1000 may also include an expander, a smoke control device, an input/output device, a network access device, etc.

The light source 10 is operable to provide an illumination light source to a site 100 to be observed. The illumination light source includes a visible light illumination light source and a laser illumination light source which is corresponding to a fluorescent reagent (e.g., near-infrared light). The light source 10 includes, but is not limited to, a laser light source, an LED light source, or a laser diode.

In the present embodiment, the light source 10 includes a visible light source and a laser light source which is corresponding to a fluorescent reagent. The visible light source is LED light source. In one embodiment, the visible light source can provide a plurality of monochromatic light in different wavelength ranges, such as blue light, green light, red light, etc. In other embodiments, the visible light source can also provide the combined light of the plurality of monochromatic lights or can be a wide spectrum white light source. The wavelength range of the monochromatic light is approximately 400 nm to 700 nm. The laser light source is operable to generate a laser light. The laser light is, for example, a near infrared (NIR) laser light. The peak wavelength of the laser light takes at least one value in the range of 780nm or 808nm.

Since the light source 10 can provide continuous visible light and laser light which is corresponding to the fluorescent reagent to the site 100 to be observed at the same time, the acquisition efficiency of the endoscope camera 50 for the visible light image signal and fluorescent image signal which are reflected by the site 100 to be observed is improved.

Before imaging with the endoscopic camera system 1000, a contrast agent, such as indocyanine green (ICG), is introduced into the site 100 to be observed by intravenous or subcutaneous injection, so as to image the tissue structure and function (such as blood/lymph/bile in the vessel) which are not easy to observe with the standard visible light imaging technology. The site 100 to be observed includes, but is not limited to, the blood circulation system, the lymphatic system and tumor tissue. ICG, commonly known as indocyanine green and diagnostic green needle, is a commonly used contrast agent in the clinical diagnosis of cardiovascular diseases. It is widely used in choroidal and retinal angiography. When the contrast agent in the site 100 to be observed absorbs the laser light generated by the laser light source corresponding to the fluorescent reagent, fluorescence light can be generated.

An endoscope camera 50 is provided in an embodiment. This disclosure takes a hard endoscope camera as an example. However, the endoscope camera can also be applied to a soft endoscope.

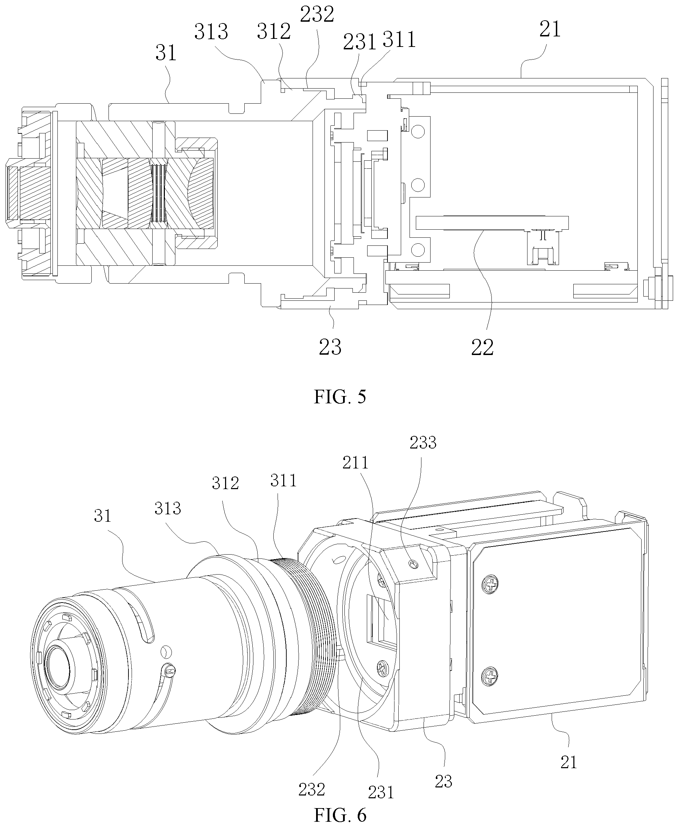

As shown in FIG.2 and FIG.6, an endoscope camera 50 of this embodiment includes a handle 1, a chip module 2, an optical module 3 and a hand wheel 4.

The handle 1 has the functions of components accommodation and holding. The handle 1 is provided with an accommodation cavity 11, wherein both ends of the handle 1 are provided with an opening which is communicated with the accommodation cavity 11. The openings at both ends of the handle 1 are connected with a communication cable 81 and the optical module 3 respectively. The chip module 2 is arranged inside the handle 1, and a button assembly 12 is also installed at the handle 1. The button assembly 12 is connected with the chip module 2 through a cable. The doctor can hold the handle 1 and control the imaging detection of the endoscopic camera through the button assembly 12. A front cover 5 is arranged at one end of the handle 1 which is adjacent to the hand wheel 4. The front cover 5 has a through hole. The front cover 5 covers the opening of the handle 1. The front cover 5 is operable to install the chip module 2 and the optical module 3 inside the accommodation cavity 11 of the handle 1.

The chip module 2 includes a housing 21 and a chip assembly 22, wherein the housing 21 is installed inside the accommodation cavity 11. The chip assembly 22 includes components such as a sensor and a processor. The sensor is an optical sensor which is operable to convert the optical signal into an electrical signal. The sensor is operable to amplify and filter the electrical signal outputted by the sensor. The processor is operable to process the electrical signal and transmit it to the camera host 60 through the communication cable 81 for further processing. One end of the housing 21 is a light inlet end which is provided with a light inlet 211, the sensor of the chip assembly 22 is aligned with the light inlet 211, the housing 21 is also provided with a wire hole, and the communication cable 81 can extend through the wire hole of the housing 21 to connect with the processor in the housing 21.

The optical module 3 includes a lens barrel 31, a fixed optical assembly 32 and an adjustable optical assembly 33, wherein the fixed optical assembly 32 is fixedly installed at one end of the lens barrel 31, and the adjustable optical assembly 33 is axially and movably installed inside the lens barrel 31. The adjustable optical assembly 33 can move relative to the fixed optical assembly 32 for adjusting the imaging focal distance.

In this embodiment, the fixed optical assembly 32 includes a fixed lens base 321 and a fixed lens assembly 322. The fixed lens base 321 is fixed inside the lens barrel 31 by a threaded connection. The fixed lens base 321 is an annular structure, the fixed lens base 321 has a cylindrical structure which is provided with an installation hole. The fixed lens assembly 322 includes two optical lenses which are fixedly installed inside the installation hole in the two optical lenses. Two lens surfaces in the axial direction of the fixed lens assembly 322 are respectively leveled with two end faces of the fixed lens base 321.

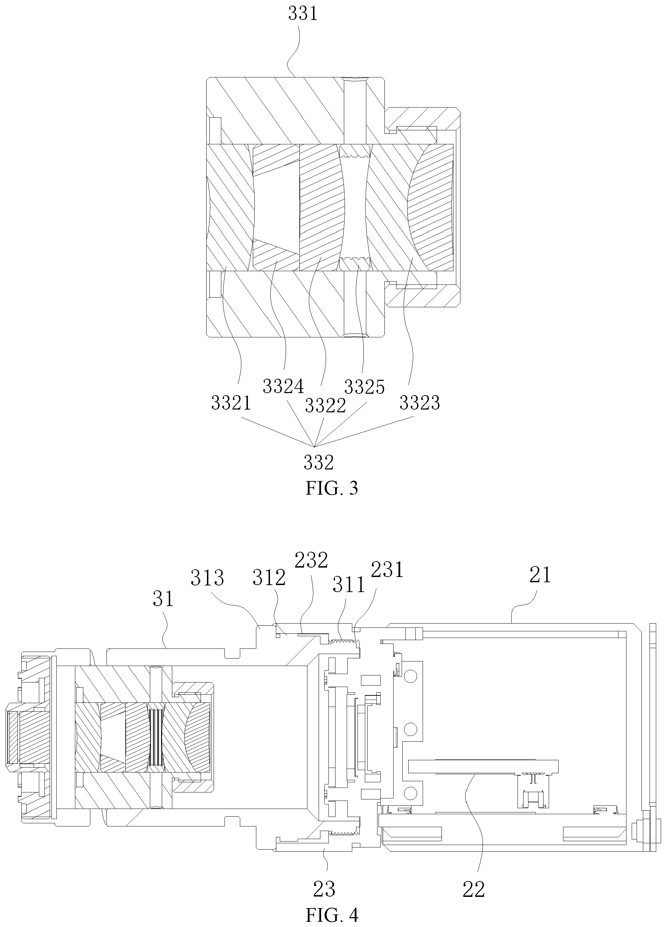

As shown in FIG. 3, the adjustable optical assembly 33 includes an adjustable lens base 331 and an adjustable lens assembly 332. The adjustable lens base 331 is slidably installed inside the lens barrel 31. The adjustable lens base 331 has a cylindrical structure with an installation hole which is coaxial with the fixed lens base 321 and the lens barrel 31. The adjustable lens assembly 332 includes a first adjustable lens 3321, a second adjustable lens 3322 and a third adjustable lens 3323, a first spacer 3324 and a second spacer 3325. The first adjustable lens 3321, the second adjustable lens 3322 and the third adjustable lens 3323 are installed inside the lens barrel 31 away from the fixed optical assembly 32 in turns, and the first spacer 3324 is installed between the first adjustable lens 3321 and the second adjustable lens 3322, the second spacer 3325 is installed between the second adjustable lens 3322 and the third adjustable lens 3323.

The first adjustable lens 3321 has a concave surface in the middle of its incident surface which faces the fixed optical assembly 32, an emitting surface of the first adjustable lens 3321 is a convex surface. The first adjustable lens 3321 is operable to convert the incident parallel light into diffuse light for emission. The incident surface of the second adjustable lens 3322 which faces the first adjustable lens 3321 is a plane, the emitting surface of the second adjustable lens 3322 is a convex surface. The second adjustable lens 3322 is operable to convert the diffuse light into parallel light. The third adjustable lens 3323 is a cemented lens for eliminating chromatic aberration. Cemented lens, also known as achromatic lens, is formed by cementing two single lenses. Its performance of polychromatic (white light) imaging is much higher than that of the single lens. Cemented lens is an achromatic lenses, which is formed by cementing a coronal glass positive lens of low dispersion together with a flint glass negative lens of high dispersion. In the design, different dispersion values and lens shapes are optimized at three wavelengths: blue (486.1nm), green (546.1nm) and red (656.3nm), so as to achieve the minimum chromatic aberration.

In one embodiment, the light inlet end of the housing 21 of the chip module 2 is provided with an installation base 23 which is protruded from the light inlet 211. The installation base 23 is fixed to the housing 21 by means of a screw or threaded connection, and the installation base 23 and the housing 21 can also be an integrated structure.

As shown in FIG.4, the installation base 23 is provided with an installation hole which is aligned with the light inlet 211 of the housing 21. The installation hole of the installation base 23 is provided inside with a first connection part 231, an outer circumference at an end of the lens barrel 31 is provided with a second connection part 311, wherein the first connection part 231 is connected with the second connection part 311. The end of the lens barrel 31 penetrates into the through hole of the front cover 5, and is inserted and installed into the installation hole of the installation base 23. The first connection part 231 is connected with the second connection part 311. In one embodiment, the first connection part 231 is an internal thread and the second connection part 311 is an external thread, so the lens barrel 31 and the installation base 23 are in a threaded connection.

As shown in FIG.5, in other embodiments, the first connection part 231 is an engaging groove, and the second connection part 311 is an engaging element. The engaging element is engaged into the engaging groove through its elastic deformation, thus forming a fixed connection. Or the first connection part 231 is an engaging element, and the second connection part 311 is an engaging groove, which can also realize the engagement between the lens barrel 31 and the installation base 23.

In one embodiment, the installation hole of the installation base 23 is further provided with a first positioning part 232 which is arranged axially with the first connection part side by side 231. The first positioning part 232 is located at an outer side along the installation direction of the installation hole, and the first connection part 231 is located at an inner side along the installation direction of the installation hole. The lens barrel 31 is provided with a second positioning part 312 which is corresponding to the first positioning part 232. The first positioning part 232 abuts against the second positioning part 312. The installation base 23 limits the radial position of the lens barrel 31 through the second positioning part 312, which is conducive to the alignment of the lens barrel 31 with the light inlet 211. In this embodiment, the first positioning part 232 is three axial protrusions which are uniformly distributed on an inner wall of the installation hole of the installation base 23, and the three axial protrusions form a three-claw position-limiting structure. The second positioning part 312 is an annular surface, and the three axial protrusions which clamp the annular surface of the lens barrel 31, are operable to limit the radial position of the lens barrel 31. The arrangement of the three axial protrusions which replaces the position-limiting structure of surface to surface, reduces the processing difficulty and processing cost of the first positioning part 232, as only it just needs to ensure the accuracy of the positioning surfaces of several axial protrusions which are in contact with the annular surface of the lens barrel 31. In other embodiments, the first positioning portion 232 is an annular surface, and the second positioning portion 312 is a plurality of axial protrusions. Alternatively, the first positioning part 232 is a plurality of axial protrusions, and the second positioning part 312 is one annular protrusion, which can also carry out the plug-in positioning of the lens barrel 31. Among them, there also can be 2 or 4, or other numbers of axial protrusions.

The light inlet end of the housing 21 is also provided with a filter, which is installed at the outside of the light inlet through a support, the end of the lens barrel 31 which connected with the housing 21 is provided with an expanded inner cavity, and the filter of the housing 21 is located in the expanded inner cavity at the end of the lens barrel 31.

The hand wheel 4 is rotatably sheathed outside the lens barrel 31. The lens barrel 31 is provided with a spiral groove. The hand wheel 4 is connected with the adjustable optical assembly 33 in the lens barrel 31 through connectors such as pins. The pins penetrate into the spiral groove of the lens barrel 31. After the hand wheel 4 rotates, under the position-limiting action of the spiral groove of the lens barrel 31, the hand wheel 4 and the adjustable optical assembly 33 will rotate and move axially at the same time, so that the hand wheel 4 can be operable to adjust the axial position of the adjustable optical assembly 33 in the lens barrel 31.

In this embodiment, the optical module 3 is directly connected with the chip module 2, the end of the lens barrel 31 of the optical module 3 is directly connected with the light inlet end of the housing 21 of the chip module 2, there are no other components between the optical module 3 and the chip module 2, the calculation of the dimension chain is shorter, the processing and installation errors are reduced, the alignment accuracy of the optical module 3 and the chip module 2, as well as, the optical imaging quality, is improved. In addition, the optical module 3 is directly connected with the chip module 2, and a part of the optical module 3 is inserted into the handle 1, which reduces the axial size of the whole endoscopic camera 50 and realizes the miniaturization of the endoscopic camera.

The direct connection in this embodiment means that there is no component, which not belongs to the optical module 3 and the chip module 2, between the optical module 3 and the chip module 2. For example, there is no front cover 5 between the optical module 3 and the chip module 2. In other words, the connection between any component of the optical module 3 and any component of the chip module 2 is a direct connection.

In this embodiment, the lens barrel 31 is provided with a position-limiting part 313 which is adjacent to and protrudes from the second positioning part 312, and the second positioning part 312 is located between the second connection part 311 and the position-limiting part 313. The position-limiting part 313 is an annular protrusion. An end face of the installation base 23, which is away from the light inlet 211, abuts against an axial side surface of the position-limiting part 313. The position-limiting part 313 is operable to locate a depth position of the lens barrel 31 which is inserted into the installation base 23, so that the position-limiting part 313 can be operable to locate the optical path size of the optical module 3 and the chip module 2. The position-limiting part 313 is located inside the accommodation cavity of the handle 1, the inner side of the front cover 5 abuts against the axial side surface of the position-limiting part 313 of the lens barrel 31, and the front cover 5 plays the role of axial position-limitation and limits the position of the end of the optical module 3 into the accommodation cavity 11 of the handle 1. In other embodiments, the position-limiting part 313 is a plurality of radial protrusions, which are uniformly distributed on an outer circumference of the lens barrel 31, and the plurality of radial protrusions can also play the role of position-limitation.

In this embodiment, the installation base 23 is provided with a locking hole, and a locking screw 233 is installed in the locking hole. The end of the locking screw 233 extends to the interior of the installation base 23 and abuts against the lens barrel 31. The locking screw 233 presses the end of the lens barrel 31 into the installation base 23, which can prevent loosening between the lens barrel 31 and the installation base 23.

In one embodiment, one end of the lens barrel 31 is inserted into the installation base 23 of the housing 2 and fixed by bonding or the like.

In one embodiment, the lens barrel 31 and the housing 2 are an integrated structure, and the lens barrel 31 and the housing 2 are processed as a whole.

In one embodiment, the lens barrel 31 can also be sheathed outside the installation base 23 of the housing 2, and the installation base 23 is provided with a cylindrical surface which is adapted to the inner surface of the lens barrel 31.

In one embodiment, one end of the lens barrel 31, which is away from the fixed optical assembly 32, is expanded, and the expanded end of the lens barrel 31 is sheathed outside the light inlet end of the housing 2. The expanded end of the lens barrel 31 is provided with an internal thread and a positioning strip, and the outer wall of the light inlet end of the housing 2 is provided with a corresponding external thread and a positioning ring. The light inlet end of the housing 2 is installed into the lens barrel 31 through a threaded connection, and is installed and positioned through the positioning strip and the positioning ring, which also realizes the direct and accurate connection between the optical module 3 and the chip module 2.

In one embodiment, the end face of the light inlet end of the housing 2 is provided with a groove, a side wall of the groove is provided with a thread, one end of the lens barrel 31 which is away from the fixed optical assembly 32 is provided with a corresponding external thread or internal thread, and one end of the lens barrel 31 is inserted and installed into the groove of the housing 2, which also realizes the direct and accurate connection between the optical module 3 and the chip module 2.

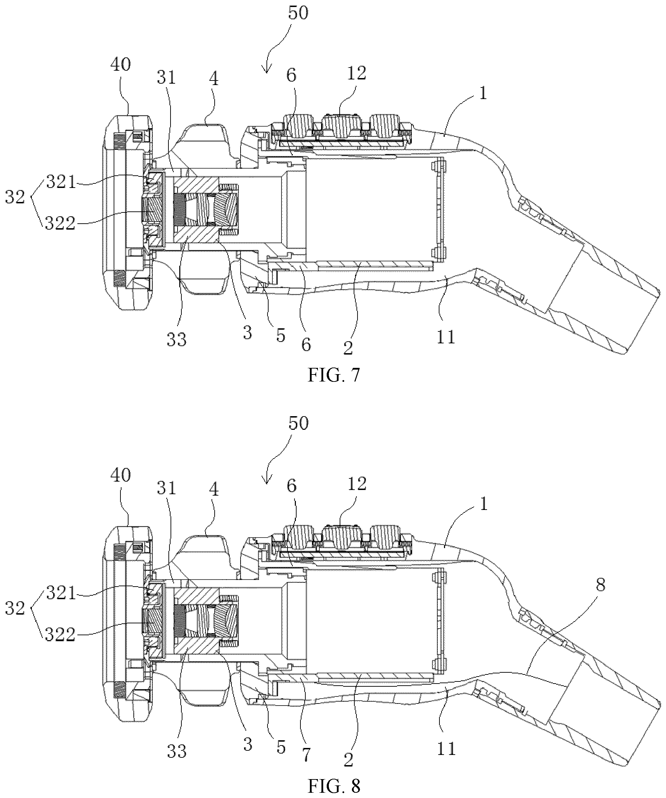

As shown in FIG.7, in one embodiment, the endoscope camera 50 further includes a fixing support 6 which is integrally positioned inside the accommodation cavity 11 of the handle 1. The fixing support 6 is operable to fix the chip module 2 on the front cover 5. The chip module 2 has a square structure, and is suspended inside the accommodation cavity of the handle 1.

The fixing support 6, which has an L-shaped structure, is formed by bending a plate. The fixing support 6 includes a long arm and a short arm, wherein the long arm of the fixing support 6 is attached to the installation base 23 and the housing 21, and the short arm and long arm of the fixing support 6 are respectively fixed with the housing 21 and the front cover 5 through screws. The fixing support 6 plays the role to fix the housing 21 on the front cover 5.

In order to achieve better fixation effect, there are two fixing supports 6. The two fixing supports 6 are installed at the upper and lower ends of the housing 21 for jointly fixing the housing 21 on the front cover 5. The fixing support 6 has an L-shaped structure and can be attached to surfaces of the front cover 5 and the housing 21, which makes the overall structure more compact and takes up less space, and is conducive to the miniaturization of the handle 1.

As shown in FIG.8, the lower fixing support 6 is a grounded support 7 which has the functions of fixing the chip module 2 and anti-static operation. The grounded support 7 is connected with the grounded cable 8. One end of the grounded cable 8 is connected with the long arm of the grounded support 7 through a screw, and the other end of the grounded cable 8 extends into the communication cable 81. The grounded support 7 can discharge the static electricity contacted by the endoscope camera 50 from the grounded cable 8, avoiding the static electricity from entering the chip assembly 2 and protecting the components in the chip assembly 2.

The end of the long arm of the grounded support 7 extends to the end of the housing 21. The grounded support 7, which has a long arm, contacts the front cover 5, the installation base 23 and the housing 21 at the same time. The grounded support 7 has a larger contact area, so that the static electricity contacted by the endoscope camera 50 during use can basically be transmitted to the grounded support 7 and then discharged from the grounded cable 8.

In other embodiments, there may be four fixing supports 6 which are respectively installed on the four surfaces of the chip module 2.

In one embodiment, one endoscope camera 50 is further provided. The difference between the endoscope camera of this embodiment and the above embodiments is that the focal distance of the optical module in the endoscope camera of this embodiment is not adjustable, and all the optical lenses are fixedly installed. Specifically, the adjustable lens base 331 is fixedly connected with the lens barrel 31.

In this embodiment, the handle 1 has the functions of components accommodation and holding. The handle 1 is provided with an accommodation cavity 11, wherein both ends of the handle 1 are provided with an opening which is communicated with the accommodation cavity 11. The openings at both ends of the handle 1 are connected with the communication cable 81 and the optical module 3 respectively. The chip module 2 is arranged inside the handle 1, and a button assembly 12 is also installed at the handle 1. The button assembly 12 is connected with the chip module 2 through a cable. The doctor can hold the handle 1 and control the imaging detection of the endoscopic camera through the button assembly 12.

The chip module 2 includes components, such as a sensor, a processor and so on. The chip module 2 is operable to convert an optical signal into an electrical signal, process the electrical signal and transmit it to the camera host 60 for imaging through the communication cable 81.

One end of the optical module 3 directly penetrates into the accommodation cavity 11 of the handle 1 to connect with the chip module 2. One end of the optical module 3 can also be connected with the chip module 2 through the front cover, and the whole optical module 3 is located outside the accommodation cavity 11 of the handle 1.

The optical module 3 includes a lens barrel 31, a fixed optical assembly 32, an adjustable optical assembly 33 and an anti-collision terminal 34. One end of the lens barrel 31 is directly installed at the opening at one end of the handle 1 which is away from the communication cable 81 or is installed at the opening at one end of the handle 1 which is away from the communication cable 81 through the front cover, and the other end of the lens barrel 31 is connected with the optical bayonet 40. The fixed optical assembly 32 and the adjustable optical assembly 33 are installed inside the lens barrel 31. The adjustable optical assembly 33 is fixed inside the lens barrel 31 through screws or pins. The adjustable optical assembly 33 has an adjustable installation. After installation, the adjustable optical assembly 33 cannot be moved. If it is necessary to adjust the installation position, it is necessary to release the locking of screws or pins, unlock and move the adjustable optical assembly 33, and then fix and lock the adjustable optical assembly 33, so as to realize the adjustable installation.

In one embodiment, an endoscope camera 50 is provided. The difference between the endoscope camera of this embodiment and the above embodiments is that the optical lens is directly fixed inside the lens barrel.

In this embodiment, the endoscope camera 50 is a camera with a fixed focal length. The endoscope camera includes a lens barrel and an optical component. The optical assembly includes several optical lenses and spacers. The optical lens includes an optical lens for emitting diffused light and parallel light. The emitting surface of the optical lens is connected with a spacer with a through hole. The shape of the through hole of the spacer is consistent with the shape of the light beam passing through it, ensuring that the stray light emitted by the optical lens can be blocked by the spacer at the rear end, but the effective light is free to pass through. In such a way, the imaging quality can be improved.

An endoscope camera 50 is provided in an embodiment. This disclosure takes a hard endoscope camera as an example. However, the endoscope camera can also be applied to a soft endoscope.

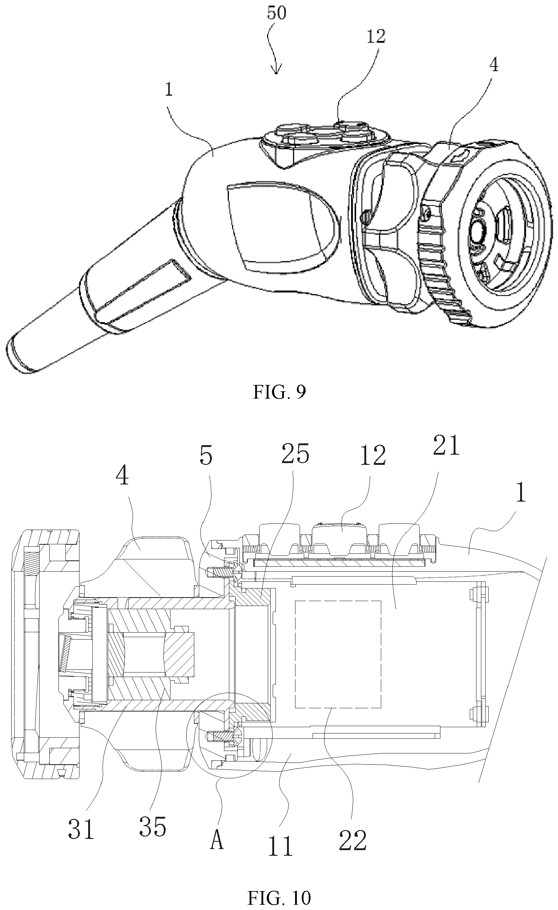

As shown in FIG.9 and FIG. 10, an endoscope camera 50 of this embodiment includes a handle 1, a chip module 2, an optical module 3 and a hand wheel 4.

The handle 1 has the functions of components accommodation and holding. The handle 1 is provided with an accommodation cavity 11, wherein both ends of the handle 1 are provided with an opening which is communicated with the accommodation cavity 11. The openings at both ends of the handle 1 are connected with a communication cable 81 and the optical module 3 respectively. The chip module 2 is arranged inside the handle 1, and a button assembly 12 is also installed at the handle 1. The button assembly 12 is connected with the chip module 2 through a cable. The doctor can hold the handle 1 and control the imaging detection of the endoscopic camera through the button assembly 12. A front cover 5 is arranged at one end of the handle 1 which is adjacent to the hand wheel 4. The front cover 5 has a through hole. The front cover 5 covers the opening of the handle 1. The front cover 5 is operable to install the chip module 2 and the optical module 3 inside the accommodation cavity 11 of the handle 1.

The chip module 2 includes a housing 21, a chip assembly 22, and an adjustment element 24. The housing 21 is installed inside the accommodation cavity 11. The chip assembly 22 includes components such as a sensor and a processor. The sensor is an optical sensor which is operable to convert the optical signal into an electrical signal. The sensor is operable to amplify and filter the electrical signal outputted by the sensor. The processor is operable to process the electrical signal and transmit it to the camera host 60 through the communication cable 81 for further processing. One end of the housing 21 which faces the front cover 5 is a light inlet end. The light inlet end of the housing 21 is provided with a light inlet 211, the sensor of the chip assembly 22 is aligned with the light inlet 211. The housing 21 is also provided with a wire hole, and the communication cable 81 can extend through the wire hole of the housing 21 to connect with the processor in the housing 21.

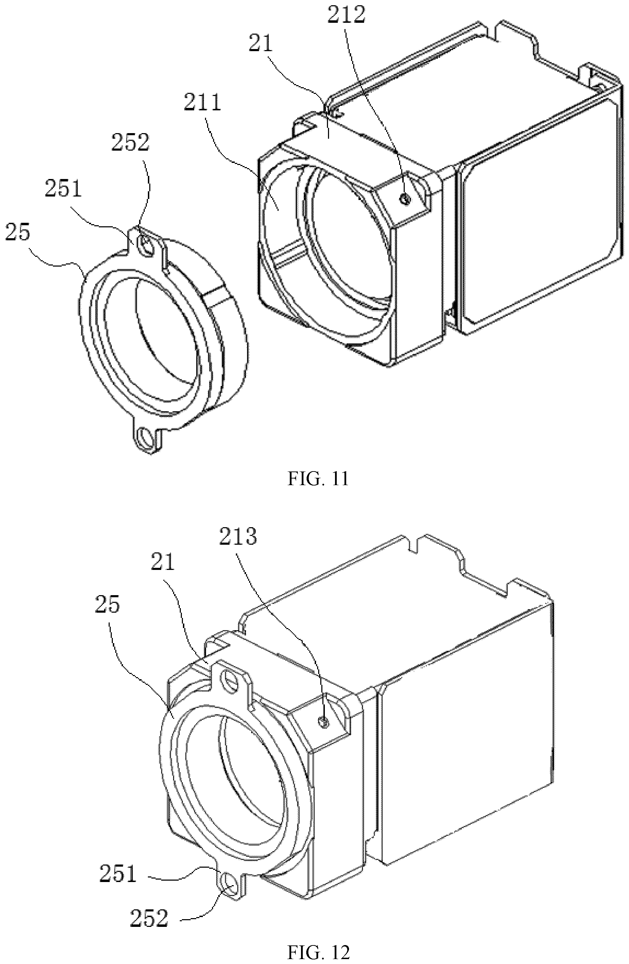

Referring FIG. 11 and FIG. 12, the light inlet end of the housing 21 is provided with an adjustment base 25 and an installation hole. The adjustment base 25 has a cylindrical structure, an end of the adjustment base 25 is inserted into the installation hole of the housing 21, the other end of the adjustment base 25 is provided with a radial convex ring, and an axial end face of the convex ring abuts against the end face of the light inlet end of the housing 21 to play the role of axial position-limitation. The installation of the adjustment base 25 can be axially adjusted relative to the housing 21. The housing 21 is provided with a screw hole 212, and a fixing screw 213 is installed inside the screw hole 212. The end of the fixing screw 213 penetrates into the screw hole 212 and abuts against an outer wall of the adjustment base 25. The fixing screw 213 locks the adjustment base 25 inside the housing 21. After loosening the fixing screw 213, the adjustment base 25 can move axially relative to the adjustment base 25, thereby adjusting the axial position of the adjustment base 25 to a preset position and then locking the adjustment base 25 at the preset position by the fixed screw 213 during the installation process, thereby improving the axial position accuracy of the chip assembly 22 and the optical module 3 and making the focusing of the chip assembly 22 more accurate

The end of the adjustment base 25, which is exposed from the housing 21, is provided with a first connection part 251 which is radially protruded. Preferably, two symmetrical first connection parts 251 are provided, and the two first connection parts 251 are respectively provided with axial first adjustment holes 252.

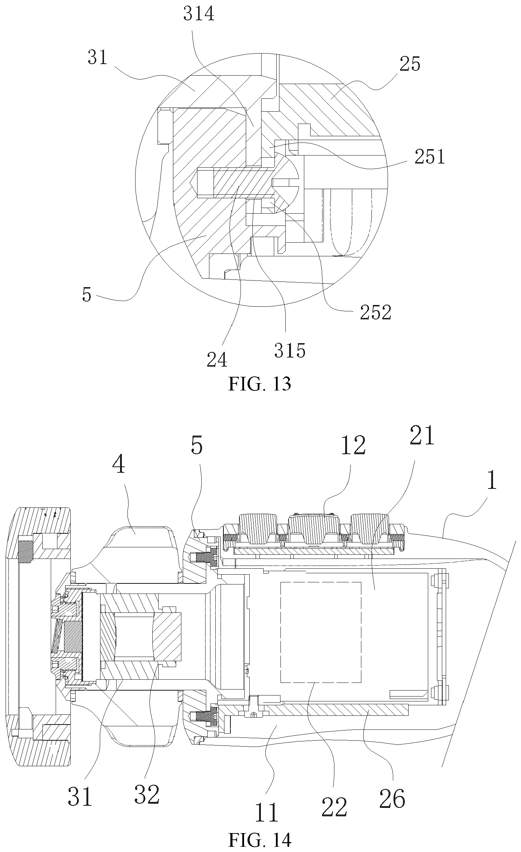

Referring to FIG. 10 and FIG. 13, the optical module 3 includes a lens barrel 31 and an optical assembly 35 which is fixedly installed inside the lens barrel 31. The optical assembly 35 includes a plurality of lenses and is operable to transmit an imaging light to the chip assembly 22. The optical assembly 35 can also be axially and adjustably installed inside the lens barrel 31. A hand wheel 4, which is sheathed outside the lens barrel 31, penetrates into the lens barrel 31 and connects with the optical assembly 35 through a connection element. In such a way, the axial movement of the optical assembly 35 can be realized by rotating the hand wheel, that is, the optical focus distance variation can be realized.

The lens barrel 31 has a light inlet end and a light outlet end, and the end of the lens barrel 31, which faces the chip module 2, is the light outlet end. An outer circumferential edge of the light outlet end of the lens barrel 31 is provided with a second connection part 314 which is radially protruded. There are two symmetrical second connection parts 314 which are respectively provided with axial second adjustment holes 315 which are correspond to the first adjustment holes 252 one by one.

Two screw holes are arranged at the axial inner side surface of the front cover 5, the light outlet end of the lens barrel 31 is inserted into the through hole of the front cover 5, the second connection part 314 of the lens barrel 31 is attached to the inner side surface of the front cover 5, the first connection part 251 of the adjustment base 25 is attached to the second connection part 314 of the lens barrel 31. The first adjustment hole 252, the second adjustment hole 315 and the screw hole of the front cover 5 are aligned with each other.

The adjustment element 24 is an adjustment rod which specifically has a screw structure. The adjustment element 24 successively penetrates into the first adjustment hole 252 of the first connection part 251 and the second adjustment hole 315 of the second connection part 314 and connects with the screw hole of the front cover 5. The adjustment element 24 locks the adjustment base 25 and the lens barrel 31 to the front cover 5, and then fixes the chip module 2 and the optical module 3 to the handle 1.

An inner diameter of the first adjustment hole 252 of the first connection part 251 is greater than an outer diameter of the adjustment element 24, and an inner diameter of the second adjustment hole 315 of the second connection part 314 is equal to or slightly greater than the outer diameter of the adjustment element 24. The first adjustment hole 252 has a gap that moves radially relative to the adjustment element 24. Then, when the adjustment element 24 is released, the radial position of the adjustment base 25 relative to the lens barrel 31 can be adjusted. When the chip assembly 22 is adjusted to be axially aligned with the optical assembly 35, the adjustment base 25 is locked and fixed through the adjustment element 24.

In this embodiment, the inner diameter of the first adjustment hole 252 is greater than the outer diameter of the adjustment element 24 to realize the radial adjustment, so that the chip assembly 22 and the optical assembly 35 can be axially aligned through the radial adjustment, thus correcting the problem of imaging eccentricity and improving the imaging quality.

In other embodiments, the inner diameter of the second adjustment hole 315 is greater than the outer diameter of the adjustment element 24, such that the chip assembly 22 and the optical assembly 35 can be axially aligned through adjusting the radial position of the lens barrel 31 relative to the adjustment base 25. Alternatively, the inner diameter of the first adjustment hole 252 and the inner diameter of the second adjustment hole 315 are greater than the outer diameter of the adjustment element 24, and the chip assembly 22 and the optical assembly 35 can also be adjusted to be axially aligned through adjusting the radial positions of the lens barrel 31 and the adjustment base 25 at the same time.

In other embodiments, the lens barrel 31 is provided with an additional radial protrusion for the fixed installation, wherein the radial protrusion is provided with a through hole, the lens barrel 31 is fixed to the front cover 5 by screws, and the adjustment element 24 is operable to lock the adjustment base 25 to the lens barrel 31. Wherein, the second adjustment hole 315 is arranged as a screw hole, and the inner diameter of the first adjustment hole 252 is greater than the outer diameter of the adjustment element 24. The adjustment element 24 locks the adjustment base 25 to the lens barrel 31 through the threaded connection with the second adjustment hole 315, which can also adjust the radial position of the adjustment base 25 relative to the lens barrel 31 and fix the lens barrel 31 and the adjustment base 25 to the front cover 5.

In one embodiment, an endoscope camera is provided. The difference between the endoscope camera of this embodiment and the above embodiments is that, the adjustment base 25 is omitted, and the housing 21 is directly connected with the lens barrel 31.

An outer circumferential edge of the light inlet end of the housing 21 is provided with two first connection parts 315, which are radially protruded. The first connection part 251 is provided with a first adjustment hole 252. The adjustment element 24 fixes the housing 21 and the lens barrel 31 to the front cover 5. As the housing 21 is provided with a first adjustment hole 252 with an inner diameter which is greater than the outer diameter of the adjustment element 24, the axial alignment of the chip assembly 22 and the optical assembly 35 can be realized through adjusting the radial position of the housing 21.

In one embodiment, an endoscope camera is provided. The difference between the endoscope camera of this embodiment and the above embodiments is that the light inlet end of the housing 21 is installed with a support 26 which replaces the adjustment base 25 to implement the axial alignment adjustment.

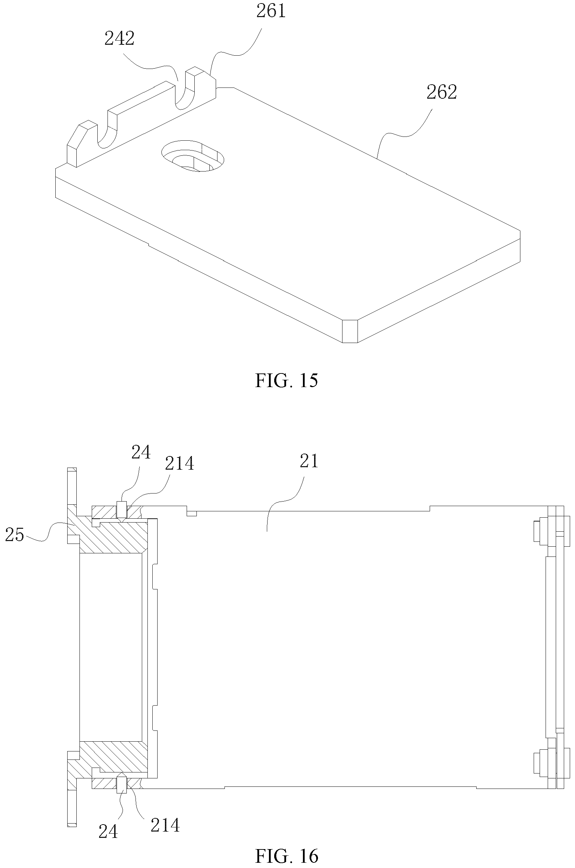

Referring to FIG. 14 and FIG. 15, in one embodiment, the existing support 26 in the endoscope camera is used as an adjustment base, and there is no need to arrange an additional adjustment base, which can save the cost. Support 26 is operable to install the housing 21 to the front cover 5. The support 26 has an L-shaped structure. The support 26 includes a first connection part 261 and a support part 262 which are perpendicular to or approximatively perpendicular to each other. The first connection part 261 is provided with a first adjustment hole 242, which is operable to install the adjustment element 24. The housing 21 is fixed to the support portion 262 of the support 26 by screws.

The adjustment element 24 is threaded to the front cover 5 after penetrating into the first adjustment hole 252 in the support 26 and the second adjustment hole 315 in the lens barrel 31. The inner diameter of the first adjustment hole 252 is greater than the outer diameter of the adjustment element 24, and then the axial alignment of the chip assembly 22 with respect to the optical assembly 35 can be adjusted by adjusting the radial position of the support 26 with respect to the lens barrel 31.

In one embodiment, an endoscope camera is provided. The difference between the endoscope camera of this embodiment and the above embodiments is that, the adjustment element 24 is operable to adjust the radial position between the housing 21 and the adjustment base 25, thus adjusting the axial alignment between the chip assembly 22 and the optical assembly 35.

Referring to FIG. 16, in this embodiment, the light outlet end of the lens barrel 31 and the adjustment base 25 are fixed to the front cover 5 by screws, and the lens barrel 31 is fixed relative to the adjustment base 25. There is no installation adjustment between them. The outer diameter of an end of the adjustment base 25 is smaller than the inner diameter of the installation hole at the light inlet end of the housing 21, and the adjustment base 25 has a space for radial movement relative to the housing 21. The light inlet end of the housing 21 is provided with at least three uniformly distributed adjustment holes 214 at one circumference, wherein the adjustment hole is a screw hole, and the adjustment element 24 is a screw or a bolt. One adjustment element 24 is installed inside each adjustment hole 214 of the housing 21, and one end of the adjustment element 24 abuts against the outer side surface of the adjustment base 25. The adjustment element 24 adjusts the radial position of the adjustment base 25 by screwing into the adjustment hole of the housing 21 in different depths, so as to adjust the alignment of the chip assembly 22 with respect to the optical assembly 35.

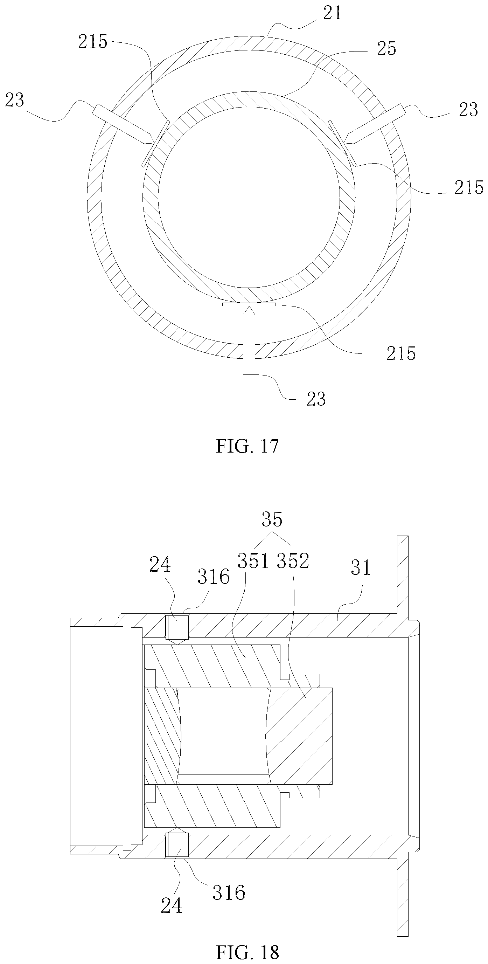

In one embodiment, referring to FIG. 17, the adjustment element 23 is installed at the housing 21, and one gasket 215 is arranged between the end of each adjustment element 23 and the outer wall of the adjustment base 24. The gasket 215 is operable to increase the contact area between the adjustment element 23 and the adjustment base 24, change the point contact between the adjustment element 23 and the adjustment base 24 into surface contact, improve the force between the adjustment element 23 and the adjustment base 24, and make the adjustment of the adjustment element 23 more accurate.

In this embodiment, the optical module 3 is fixedly installed, and the radial position of the chip assembly 22 inside the chip module 2 is adjusted separately to realize the alignment adjustment with the optical assembly 35.

In one embodiment, an endoscope camera is provided. The difference between the endoscope camera of this embodiment and the above embodiments is that, the adjustment element 24 belongs to a part of the optical module 3, and the adjustment base 25 is installed at the lens barrel 31.

Referring to FIG. 18, in this embodiment, the optical module 3 includes a lens barrel 31, an optical assembly35 and an adjustment element 24. An end of the adjustment base 25 is inserted into the light outlet end of the lens barrel 31, and the adjustment base 25 can also be adjusted and fixed axially relative to the lens barrel 31. A screw hole is arranged at the lens barrel 31, and a fixing screw is installed inside the screw hole. The end of the fixing screw extends into the lens barrel 31 and abuts against the outer wall of the adjustment base 25 to lock the adjustment base 25.

The adjustment base 25 is provided with a first connection part 251, the outer circumferential edge of the light inlet end of the housing 21 is provided with two second connection parts 314 which are radially protruded. The second connection part 314 is provided with an axial second adjustment hole 315. The inner diameter of the second adjustment hole 315 is greater than the outer diameter of the adjustment element 24, which successively fixes the housing 21 and the adjustment base 25 to the front cover 5.

Similarly, the inner diameter of the first adjustment hole 252 which is provided at the first connection part 251 is greater than the outer diameter of the adjustment element 24, or the inner diameters of the first adjustment hole 252 and the second adjustment hole 315 are greater than the outer diameter of the adjustment element 24, which can also realize the axial alignment adjustment of the chip assembly 22 with the optical assembly 35.

In one embodiment, an endoscope camera 50 is provided. The difference between the endoscope camera of this embodiment and the above embodiments is that the adjustment element 24 is operable to adjust the radial position between the optical assembly 35 and the lens barrel 31, thus adjusting the axial alignment between the chip assembly 22 and the optical assembly 35.

In this embodiment, the lens barrel 31 and the housing 21 are fixed to the front cover 5 by screws. The lens barrel 31 and the housing 21 are relatively fixed and cannot be adjusted radially. The optical assembly 35 includes a lens base 351 and a lens group 352. The lens base 351 has a cylindrical structure. The lens group 352 includes several lenses, and the lens group 352 is fixedly installed inside the lens base 351. The outer diameter of the lens base 351 is smaller than the inner diameter of the lens barrel 31, and the lens base 351 has a space for radial movement relative to the lens barrel 31.

At least three adjustment holes 316 are arranged at one circumference of the lens barrel 31, wherein the adjustment hole 316 is a screw hole, and the adjustment element 24 is a screw or a bolt. One adjustment element 24 is installed inside each adjustment hole 316 of the lens barrel 31, and one end of the adjustment element 24 abuts against the outer side surface of the lens base 351. The adjustment element 24 can adjust the radial position of the lens base 351 by screwing into the adjustment hole of the lens barrel 31 in different depths, so as to realize the axial alignment adjustment of the optical assembly 35 with respect to the chip assembly 22.

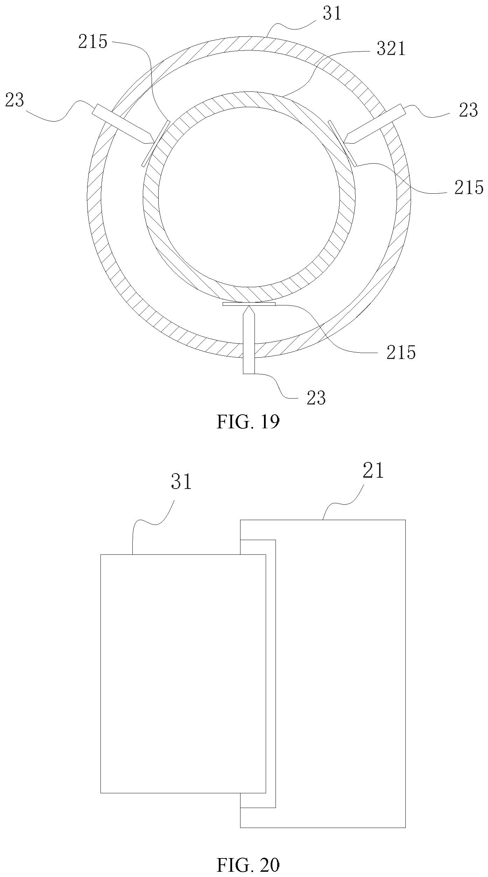

In one embodiment, referring to FIG. 19, an adjustment element 23 is installed at the lens barrel 31, and a gasket 215 is arranged between an end of each adjustment element 23 and an outer wall of the lens base 321. The gasket 215 is operable to increase a contact area between the adjustment element 23 and the lens base 321, change the point contact between the adjustment element 23 and the lens base 321 into surface contact, improve the force between the adjustment element 23 and the lens base 321, and make the adjustment of the adjustment element 23 more accurate.

In an embodiment, an endoscope camera is provided, which includes at least two above adjustment modes. For example, an adjustment element 24 is installed at the lens barrel 31, and an adjustment element 24 is also installed between the lens barrel 31 and the housing 21. Among them, the adjustment element 24 at the lens barrel 31 is operable to adjust the radial position of the optical assembly 35 and the lens barrel 31, and the adjustment element 24 between the lens barrel 31 and the housing 21 is operable to adjust the radial position of the housing 21 relative to the lens barrel 31. Both adjustment modes can independently realize the axial alignment adjustment of the chip assembly 22 and the optical assembly 35, while when the two adjustment methods coexist, the axial alignment adjustment of the chip assembly 22 and the optical assembly 35 can be more efficiently.

In an embodiment, an endoscope camera is provided. The difference between the endoscope camera of this embodiment and the above embodiments is that the housing 21 of the chip module 20 and the lens barrel 31 of the optical module 30 are directly connected together in a radially adjustable manner.

Referring to FIG. 20, one end of the housing 21, which faces the lens barrel 31, is provided with an installation hole, and the end of the lens barrel 31 is inserted into the installation hole of the housing 21. The inner diameter of the installation hole is slightly greater than the outer diameter of the end of the lens barrel 31, so that there is a radial adjustment gap between the lens barrel 31 and the installation hole of the housing 21.

In one embodiment, during the installation process, after the end of the lens barrel 31 is aligned with the installation hole of the housing 21, a fixing glue is infiltrated between the lens barrel 31 and the installation hole of the housing 21 to fix the lens barrel 31 and the housing 21.

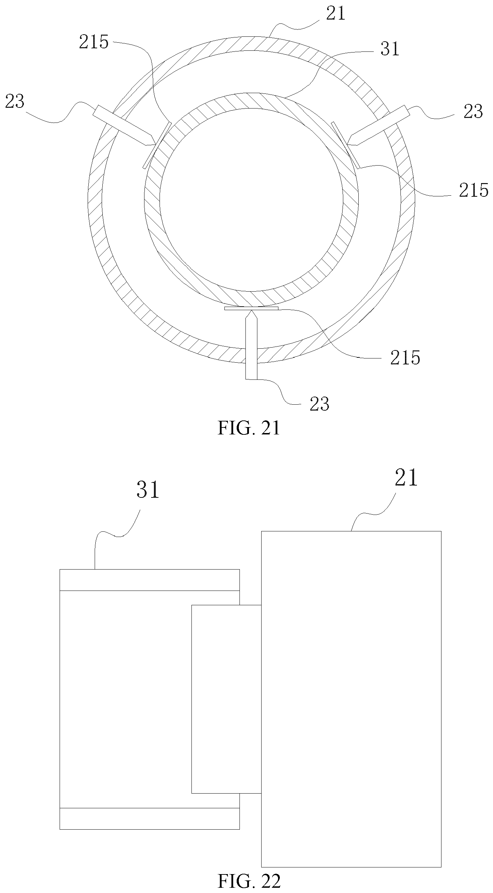

Referring to FIG. 21, in one embodiment, a plurality of adjustment holes are arranged at the housing 21, and an adjustment element 23 is installed inside each adjustment hole. The adjustment element 23 is a screw or bolt, and the adjustment hole is a screw hole. The end of the adjustment element 23 abuts against the outer circumferential surface of the lens barrel 31. Moreover, a gasket 215 is arranged between the end of the adjustment element 23 and the lens barrel 31. The gasket 215 is operable to increase the contact area between the adjustment element 23 and the lens barrel 31, change the point contact between the adjustment element 23 and the lens barrel 31 into surface contact, improve the force between the adjustment element 23 and the lens barrel 31, and make the adjustment of the adjustment element 23 more accurate.

In an embodiment, an endoscope camera is provided. The difference between the endoscope camera of this embodiment and the above embodiments is that the housing 21 of the chip module 20 and the lens barrel 31 of the optical module 30 are directly connected together in a radial adjustable manner.

Referring to FIG. 22, a cylindrical installation part is arranged at one end of the housing 21 towards the lens barrel 31. The installation part of the housing 21 is inserted into one end of the lens barrel 31. The inner diameter of the lens barrel 31 is slightly greater than the outer diameter of the installation part of the housing 21, so that there is a radial adjustment gap between the lens barrel 31 and the installation part of the housing 21.

In one embodiment, in the installation process, after the installation part of the housing 21 is aligned with the lens barrel 31, a fixing glue is infiltrated between the installation part of the housing 21 and the lens barrel 31 to fix the lens barrel 31 and the housing 21.

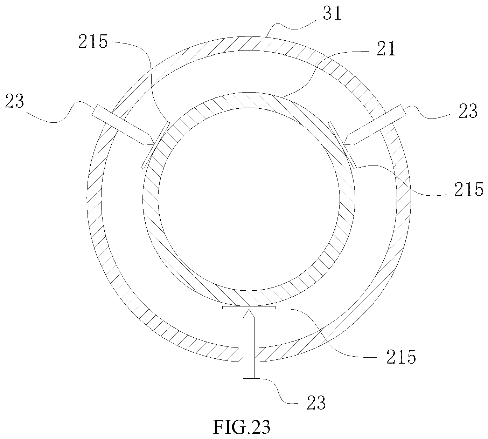

Referring to FIG. 23, in one embodiment, a plurality of adjustment holes are arranged at the lens barrel 31, and one adjustment element 23 is installed inside each adjustment hole. The adjustment element 23 is a screw or a bolt, and the adjustment hole is a screw hole. The end of the adjustment element 23 abuts against the outer circumferential surface of the installation part of the housing 21. Moreover, a gasket 215 is arranged between the end of the adjustment element 23 and the installation part of the housing 21. The gasket 215 is operable to increase the contact area between the adjustment element 23 and the installation part of the housing 21, change the point contact between the adjustment element 23 and the installation part of the housing 21 into surface contact, improve the force between the adjustment element 23 and the installation part of the housing 21, and make the adjustment of the adjustment element 23 more accurate.

The above description is only the specific embodiments of the present disclosure, and the present disclosure is not limited to these specific embodiments. Those skilled in the art can make various changes and modifications to the present disclosure without departing from the spirit and scope of the present disclosure. Obviously, these changes and variants should fall within the protection scope required by this present disclosure. In addition, although some specific terms are used in this disclosure, however these terms are only for convenience of explanation and do not constitute any special restrictions to this disclosure.

## Claims

1. An endoscope camera, **characterized in that**, comprising:
a handle which is provided with an accommodation cavity, wherein an end of the handle is provided with an opening which is communicated with the accommodation cavity;
a chip module which comprises a housing and a chip assembly, wherein the housing is installed inside the accommodation cavity of the handle, the chip assembly is installed inside the housing, wherein the housing is provided with a light inlet end at which a light inlet is arranged, wherein the chip assembly is operable to convert an optical signal into an electrical signal;
an optical module which is connected with the chip module, wherein the optical module comprises a lens barrel, a fixed optical assembly and an adjustable optical assembly, wherein the fixed optical assembly is installed at one end of the lens barrel which is away from the chip module, and the adjustable optical assembly is axially and movably installed inside the lens barrel; and
a hand wheel which is rotatably sheathed outside the lens barrel and connected with the adjustable optical assembly through a connection element, wherein the hand wheel is operable to adjust an axial position of the adjustable optical assembly.

2. The endoscope camera according to claim 1, **characterized in that**, the other end of the lens barrel is connected with the light inlet end of the housing.

3. The endoscope camera according to claim 1 or claim 2, **characterized in that**, the light inlet end of the housing is provided with an installation base, wherein the installation base is provided with an installation hole into which the other end of the lens barrel is inserted, wherein the lens barrel is axially aligned with the light inlet.

4. The endoscope camera according to claim 3, **characterized in that**, a first connection part is provided inside the installation hole of the installation base, a second connection part is provided at the other end of the lens barrel, wherein the first connection part is connected with the second connection part.

5. The endoscope camera according to claim 4, **characterized in that**, the first connection part is an internal thread and the second connection part is an external thread; or the first connection part is an engaging groove, and the second connection part is an engaging element.

6. The endoscope camera according to claim 4, **characterized in that**, the installation hole of the installation base is further provided with a first positioning part which is arranged axially with the first connection part side by side, the other end of the lens barrel is provided with a second positioning part which is arranged axially with the second connection part side by side, and the first positioning part is positionally limited on the second positioning part.

7. The endoscope camera according to claim 6, **characterized in that**, the first positioning part is a plurality of axial protrusions, and the second positioning part is an annular surface; or the first positioning part is an annular surface, and the second positioning part is a plurality of axial protrusions.

8. The endoscope camera according to claim 3, **characterized in that**, the installation base is provided with a locking hole, inside which a locking screw is installed, and the locking screw locks the other end of the lens barrel inside the installation base.

9. The endoscope camera according to claim 1, **characterized in that**, the lens barrel of the optical module and the housing of the chip module are an integrated structure.

10. The endoscope camera according to any one of claims 3-8, **characterized in that**, the opening, at the end of the handle which is adjacent to the hand wheel, is provided with a front cover, wherein the front cover is provided with a through hole, wherein the lens barrel penetrates into and is arranged inside the through hole, the housing is connected with the front cover, and the front cover is operable to install the chip module and the optical module into the accommodation cavity of the handle.

11. The endoscope camera according to claim 10, **characterized in that**, the lens barrel is provided with a position-limiting part which is operable to limit an axial position of the lens barrel.

12. The endoscope camera according to claim 11, **characterized in that**, the position-limiting part is clamped between the front cover and the installation base.

13. The endoscope camera according to claim 11, **characterized in that**, the position-limiting part is an annular protrusion or a plurality of radial protrusions arranged at a same circumference.

14. The endoscope camera according to claim 10, **characterized in that**, the endoscope camera further comprises a fixing support which is positioned inside the accommodation cavity of the handle, one end of the fixing support is connected with the front cover, and the other end of the fixing support is connected with the housing of the chip module.

15. The endoscope camera according to claim 14, **characterized in that**, the fixing support has an L-shaped structure and comprises a long arm and a short arm which are perpendicular to each other, the long arm is attached to the installation base and the housing and connected with the housing, and the short arm is attached to the front cover and connected with the front cover.

16. The endoscope camera according to claim 15, **characterized in that**, the long arm and the short arm of the fixing support are respectively fixed with the housing and the front cover through a screw.

17. The endoscope camera according to claim 15, **characterized in that**, the endoscope camera comprises a plurality of fixing supports, and at least one of the plurality of fixing supports is connected with a grounded cable.

18. The endoscope camera according to claim 17, **characterized in that**, the fixing support, which is connected with the grounded cable, is a grounded support, and an end of the long arm of the grounded support extends to an axial end of the housing.

19. An endoscope camera, **characterized in that**, comprising:
a handle which is provided with an accommodation cavity, wherein an end of the handle is provided with an opening which is communicated with the accommodation cavity;
a chip module which is installed inside the accommodation cavity of the handle and operable to convert an optical signal into an electrical signal;
an optical module which is directly connected with the chip module and exposed entirely or partially from an opening of the handle; and
a hand wheel which is rotatably sheathed outside the optical module.

20. The endoscope camera according to claim 19, **characterized in that**, the chip module is provided with an installation base, wherein the installation base is provided with an installation hole into which an end of the optical module is inserted and installed.

21. The endoscope camera according to claim 20, **characterized in that**, a first connection part is provided inside the installation hole of the installation base, a second connection part is provided at the end of the optical module, wherein the first connection part is connected with the second connection part.

22. The endoscope camera according to claim 21, **characterized in that**, the installation hole of the installation base is further provided with a first positioning part which is arranged axially with the first connection part side by side, the end of the optical module is provided with a second positioning part which is arranged axially with the second connection part side by side, and the first positioning part is positionally limited on the second positioning part.

23. An endoscope camera, **characterized in that**, comprising:
a handle which is provided with an accommodation cavity, wherein an end of the handle is provided with an opening which is communicated with the accommodation cavity;
a chip module which comprises a housing and a chip assembly, wherein the housing is installed inside the accommodation cavity of the handle, the chip assembly is installed inside the housing, wherein the housing is provided with a light inlet end at which a light inlet is arranged; and
an optical module which comprises a lens barrel and an optical assembly, wherein an end of the lens barrel is connected with the light inlet end of the housing, and the optical assembly is installed inside the lens barrel and operable to transmit an optical signal.

24. The endoscope camera according to claim 23, **characterized in that**, the housing is provided with an installation base, wherein the installation base is provided with an installation hole into which the end of the lens barrel is inserted.

25. The endoscope camera according to claim 24, **characterized in that**, a first connection part is provided inside the installation hole of the installation base, a second connection part is provided with the end of the lens barrel, wherein the first connection part is connected with the second connection part.

26. The endoscope camera according to claim 25, **characterized in that**, the installation hole of the installation base is further provided with a first positioning part which is arranged axially with the first connection part side by side, the end of the lens barrel is provided with a second positioning part which is arranged axially with the second connection part side by side, and the first positioning part is positionally limited on the second positioning part.

27. The endoscope camera according to claim 23, **characterized in that**, the chip module further comprises an adjustment element, which is arranged at the housing, for a radial adjustment of the chip assembly.

28. The endoscope camera according to claim 27, **characterized in that**, the adjustment element comprises an adjustment rod, the housing and/or the lens barrel are provided with an adjustment hole, wherein the adjustment rod penetrates into and is arranged inside the adjustment hole, and the adjustment rod is operable to adjust and fix a radial position of the chip assembly and/or a radial position of the optical assembly.

29. The endoscope camera according to claim 28, **characterized in that**, the light inlet end of the housing is provided with a first connection part, and a light outlet end of the lens barrel is provided with a second connection part; the first connection part is provided with a first adjustment hole, and the second connection part is provided with a second adjustment hole; the adjustment rod is arranged inside the first adjustment hole and the second adjustment hole and operable to fix the first connection part and the second connection part; wherein an inner diameter of the first adjustment hole is adapted to an outer diameter of the adjustment rod, an inner diameter of the second adjustment hole is greater than the outer diameter of the adjustment rod; or an inner diameter of the second adjustment hole is adapted to the outer diameter of the adjustment rod, and an inner diameter of the first adjustment hole is greater than the outer diameter of the adjustment rod; or inner diameters of both the first adjustment hole and the second adjustment hole are greater than the outer diameter of the adjustment rod.

30. The endoscope camera according to claim 28, **characterized in that**, the light inlet end of the housing is provided with an adjustment base; wherein the adjustment base is provided with a first connection part, and a light outlet end of the lens barrel is provided with a second connection part; the first connection part is provided with a first adjustment hole, and the second connection part is provided with a second adjustment hole; the adjustment rod is arranged inside the first adjustment hole and the second adjustment hole and operable to fix the first connection part and the second connection part; wherein an inner diameter of the first adjustment hole is adapted to an outer diameter of the adjustment rod, an inner diameter of the second adjustment hole is greater than the outer diameter of the adjustment rod; or an inner diameter of the second adjustment hole is adapted to the outer diameter of the adjustment rod, and an inner diameter of the first adjustment hole is greater than the outer diameter of the adjustment rod; or inner diameters of both the first adjustment hole and the second adjustment hole are greater than the outer diameter of the adjustment rod.

31. The endoscope camera according to claim 29, **characterized in that**, the light inlet end of the housing is provided with an installation hole, and an end of the adjustment base can be axially and adjustably installed inside the installation hole of the housing.

32. The endoscope camera according to claim 31, **characterized in that**, the housing is provided with a fixing screw which fixes the end of the adjustment base to the housing.

33. The endoscope camera according to claim 28, **characterized in that**, a front cover is installed at an opening of the accommodation cavity of the handle, a through hole is arranged at a middle of the front cover, and the lens barrel penetrates into and is arranged inside the through hole; wherein an axial inner side surface of the front cover is provided with a screw hole, the adjustment rod is a bolt or a screw, and the adjustment rod is connected inside the screw hole of the front cover.

34. The endoscope camera according to claim 28, **characterized in that**, the light inlet end of the housing is provided with an installation hole and an adjustment base; wherein an end of the adjustment base is installed inside the installation hole of the housing and has an outer diameter which is smaller than an inner diameter of the installation hole of the housing; wherein at least three adjustment holes are arranged at one circumference of the light inlet end of the housing, and each adjustment hole is provided with one adjustment rod.

35. The endoscope camera according to claim 34, **characterized in that**, the adjustment hole is a screw hole, the adjustment rod is a bolt or a screw, and an end of the adjustment rod penetrates into the adjustment hole and abuts against the end of the adjustment base.

36. The endoscope camera according to claim 35, **characterized in that**, a gasket is arranged between the end of the adjustment rod and the adjustment base.

37. The endoscope camera according to claim 23, **characterized in that**, the optical module further comprises an adjustment element, which is arranged at the lens barrel, for a radial adjustment of the chip assembly.

38. The endoscope camera according to claim 37, **characterized in that**, the adjustment element comprises an adjustment rod, the housing and/or the lens barrel are provided with an adjustment hole, wherein the adjustment rod penetrates into and is arranged inside the adjustment hole, and the adjustment rod is operable to adjust and fix a radial position of the chip assembly and/or a radial position of the optical assembly.

39. The endoscope camera according to claim 38, **characterized in that**, the light inlet end of the housing is provided with a first connection part, and a light outlet end of the lens barrel is provided with a second connection part; the first connection part is provided with a first adjustment hole, and the second connection part is provided with a second adjustment hole; the adjustment rod is arranged inside the first adjustment hole and the second adjustment hole and operable to fix the first connection part and the second connection part; wherein an inner diameter of the first adjustment hole is adapted to an outer diameter of the adjustment rod, an inner diameter of the second adjustment hole is greater than the outer diameter of the adjustment rod; or an inner diameter of the second adjustment hole is adapted to the outer diameter of the adjustment rod, and an inner diameter of the first adjustment hole is greater than the outer diameter of the adjustment rod; or inner diameters of both the first adjustment hole and the second adjustment hole are greater than the outer diameter of the adjustment rod.

40. The endoscope camera according to claim 38, **characterized in that**, a light outlet end of the lens barrel is provided with an adjustment base; wherein the adjustment base is provided with a first connection part, and a light inlet end of the housing is provided with a second connection part; the first connection part is provided with a first adjustment hole along its circumference, and the second connection part is provided with a second adjustment hole; an inner diameter of one of the first adjustment hole and the second adjustment hole is adapted to an outer diameter of the adjustment rod, and an inner diameter of the other one of the first adjustment hole and the second adjustment hole is greater than the outer diameter of the adjustment rod; or inner diameters of both the first adjustment hole and the second adjustment hole are greater than the outer diameter of the adjustment rod; wherein the adjustment rod is arranged inside the first adjustment hole and the second adjustment hole and operable to fix the first connection part and the second connection part.

41. The endoscope camera according to claim 38, **characterized in that**, a front cover is installed at an opening of the accommodation cavity of the handle, a through hole is arranged at a middle of the front cover, and the lens barrel penetrates into and is arranged inside the through hole; wherein an axial inner side surface of the front cover is provided with a screw hole, the adjustment rod is a bolt or a screw, and the adjustment rod is connected inside the screw hole of the front cover.

42. The endoscope camera according to claim 38, **characterized in that**, an outer diameter of the optical assembly is smaller than an inner diameter of the lens barrel, at least three adjustment holes are arranged at one circumference of the lens barrel, each adjustment hole is provided with one adjustment rod, the adjustment hole is a screw hole, the adjustment rod is a bolt or a screw, an end of the adjustment rod penetrates into the adjustment hole and abuts against the optical assembly.

43. The endoscope camera according to claim 42, **characterized in that**, the optical assembly comprises a lens base and a lens group, wherein the lens base has a cylindrical structure, the lens group is installed inside the lens base, and the end of the adjustment rod abuts against an outer circumferential surface of the lens base.

44. The endoscope camera according to claim 43, **characterized in that**, a gasket is arranged between the end of the adjustment rod and the outer circumferential surface of the lens base.

45. An endoscope camera, **characterized in that**, comprising:
a handle which is provided with an accommodation cavity, wherein an end of the handle is provided with an opening which is communicated with the accommodation cavity;
a chip module which comprises a housing and a chip assembly, wherein the housing is installed inside the accommodation cavity of the handle, the chip assembly is installed inside the housing, wherein the housing is provided with a light inlet end at which a light inlet is arranged, wherein the chip assembly is operable to convert an optical signal into an electrical signal; and
an optical module which comprises a lens barrel and an optical assembly, wherein the optical assembly is installed inside the lens barrel, and the lens barrel is provided with a light outlet end which is connected with the light inlet end of the housing;
an end of the housing, which faces the lens barrel, is provided with an installation hole, an end of the lens barrel is arranged inside the installation hole; wherein an installation gap is provided for radial adjustment between the housing and the lens barrel.

46. The endoscope camera according to claim 45, **characterized in that**, the housing and the lens barrel are fixed by glue or screw.

47. The endoscope camera according to claim 46, **characterized in that**, the housing is provided with a screw hole, the screw is fixed inside the screw hole of the housing, an end of the screw abuts against an outer circumferential surface of the lens barrel, and a gasket is arranged between the end of the screw and the outer circumferential surface of the lens barrel.

48. An endoscope camera, **characterized in that**, comprising:
a handle which is provided with an accommodation cavity, wherein an end of the handle is provided with an opening which is communicated with the accommodation cavity;
a chip module which comprises a housing and a chip assembly, wherein the housing is installed inside the accommodation cavity of the handle, the chip assembly is installed inside the housing, wherein the housing is provided with a light inlet end at which a light inlet is arranged, wherein the chip assembly is operable to convert an optical signal into an electrical signal; and
an optical module which comprises a lens barrel and an optical assembly, wherein the optical assembly is installed inside the lens barrel, the lens barrel is provided with a light outlet end which is connected with the light inlet end of the housing;
an end of the housing, which faces the lens barrel, is provided with an installation part, the installation part of the housing is arranged inside the lens barrel, and an installation gap is provided for radial adjustment between the installation part of the housing and the lens barrel.

49. The endoscope camera according to claim 48, **characterized in that**, the installation part of the housing and the lens barrel are fixed by glue or screw.

50. The endoscope camera according to claim 49, **characterized in that**, the lens barrel is provided with a screw hole, the screw is fixed inside the screw hole of the lens barrel, an end of the screw abuts against the installation part of the housing, and a gasket is arranged between the end of the screw and the installation part of the housing.

51. An endoscope camera system, **characterized in that**, comprising a light source, light guide bundles, an endoscope, an optical bayonet, a communication cable, a camera host, a display, a video connection line and the endoscope camera according to any one of claims 1-50; wherein the light source is connected with the endoscope through the light guide bundles, and one end of the endoscope camera is connected with the endoscope through the optical bayonet, the other end of the endoscope camera is connected with the camera host through the communication cable, wherein the camera host is connected with the display through the video connection cable.
